# EUROPÄISCHE PATENTSCHRIFT

(11) **EP 2 482 927 B1**
(45) Veröffentlichungstag und Bekanntmachung des Hinweises auf die Patenterteilung: **18.02.2015**
(21) Anmeldenummer: 10751632.0
(22) Anmeldetag: 31.08.2010
(51) Int. Cl.: A61N 5/10

(54) **VERFAHREN ZUR ERSTELLUNG EINES BESTRAHLUNGSPLANS, VERFAHREN ZUR ANPASSUNG UND OPTIMIERUNG EINER BESTRAHLUNGSPLANUNG, BESTRAHLUNGSPLANUNGSEINRICHTUNG SOWIE BESTRAHLUNGSANLAGE**
METHOD FOR DRAWING UP AN IRRADIATION PLAN, METHOD FOR ADAPTING AND OPTIMISING AN IRRADIATION PLANNING SYSTEM, IRRADIATION PLANNING DEVICE AND IRRADIATION INSTALLATION
PROCÉDÉ POUR ÉLABORER UN PROGRAMME DE TRAITEMENT PAR RAYONNEMENT, PROCÉDÉ POUR ADAPTER ET OPTIMISER UN PROGRAMME DE TRAITEMENT PAR RAYONNEMENT, DISPOSITIF DE PROGRAMMATION DE TRAITEMENT PAR RAYONNEMENT ET INSTALLATION DE TRAITEMENT PAR RAYONNEMENT

(30) Priorität: 30.09.2009 DE 102009043548
(43) Veröffentlichungstag der Anmeldung: 08.08.2012
(73) Patentinhaber: GSI Helmholtzzentrum für Schwerionenforschung GmbH, 64291 Darmstadt (DE)
(72) Erfinder: BERT, Christoph, 63741 Aschaffenburg (DE); GEMMEL, Alexander, 55118 Mainz (DE); RIETZEL, Eike, 64331 Weiterstadt (DE)
(74) Vertreter: Mergel, Volker
(86) Internationale Anmeldenummer: PCT/EP2010/062700
(87) Internationale Veröffentlichungsnummer: WO 2011/039022

(56) Entgegenhaltungen:
- WO-A1-2008/013956
- DE-A1-102007 045 879
- US-A1- 2003 091 146

## Beschreibung

Die Erfindung betrifft ein Verfahren zur Erstellung eines Bestrahlungsplans, ein Verfahren zur Anpassung einer Bestrahlungsplanung an eine Änderung eines Zielvolumens sowie ein Verfahren zur Optimierung einer Bestrahlungsplanung.
Weiterhin betrifft die Erfindung eine Bestrahlungsplanungseinrichtung zur Durchführung eines derartigen Verfahrens und eine Bestrahlungsanlage mit einer derartigen Bestrahlungsplanungseinrichtung. Derartige Verfahren bzw. Vorrichtungen werden insbesondere im Rahmen der Partikeltherapie eingesetzt, bei der eine vergleichsweise komplexe Bestrahlungsplanung auftritt.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nichttherapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Zielvolumen mit dem Partikelstrahl bestrahlt.

Es ist in der Partikeltherapie bekannt, eine Bestrahlung mit Ionen durchzuführen, die schwerer sind als Protonen. Bei derartigen Ionen wird von einer erhöhten biologischen Wirksamkeit ausgegangen. Dies bedeutet, dass die absorbierte Dosis ortsabhängig mit einem Faktor gewichtet wird, um die biologisch wirksame (biologische effektive) Dosis zu ermitteln. Eine derartige Dosis ist auch unter der Bezeichnung "RBW-gewichtete Dosis" bekannt. Im Allgemeinen wird die erhöhte biologische Wirksamkeit in der Bestrahlungsplanung berücksichtigt, indem der Bestrahlungsplanung ein entsprechendes Modell zu Grunde liegt, das die komplexe Interaktion des Partikelstrahls mit der Materie beschreibt.

Ziel der Bestrahlungsplanung ist es im Allgemeinen, eine zu erfüllende Dosisverteilung vorzugeben und darauf basierend die notwendigen Maschinenparameter zu bestimmen. Die Ergebnisse der Bestrahlungsplanung werden üblicherweise in einem Bestrahlungsplanungsdatensatz hinterlegt, der dann verwendet wird, wenn die tatsächliche Bestrahlung durchgeführt werden soll, um die Bestrahlungsanlage derart zu steuern, dass die zu erreichende Dosisverteilung auch bestmöglich appliziert wird.

Aufgrund der Vielzahl der zu bestimmenden Maschinenparameter werden üblicherweise Verfahren der inversen Bestrahlungsplanung verwendet. Dies bedeutet, dass die zu erreichende Dosisverteilung vorgegeben wird, und die Maschinenparameter anhand eines Optimierungsverfahrens derart bestimmt werden, dass die zu erzielende Dosisverteilung bei der Bestrahlung bestmöglich erreicht wird.

Im Dokumenten WO 2008/013956 sowie DE 10 2007 045 879 werden Korrektur Parameter separat gespeichert. Insbesondere im Rahmen der Partikeltherapie und insbesondere bei Partikeltherapieanlagen, bei denen ein Scanverfahren eingesetzt wird, ist die Bestrahlungsplanung aufgrund der Vielzahl der zu bestimmenden Maschinenparameter und aufgrund der Komplexität des Modells, das der Optimierung zu Grunde liegt, mitunter rechenaufwändig.

Es ist daher die Aufgabe der Erfindung, ein Verfahren zur Erstellung einer Bestrahlungsplanung anzugeben, das eine schnellere Berechnung und eine schnelle Durchführung einer darauf aufbauenden Bestrahlungsplanung ermöglicht.

Die Aufgabe der Erfindung wird gelöst durch die Merkmale der unabhängigen Ansprüche. Vorteilhafte Weiterbildungen finden sich in den Merkmalen der abhängigen Ansprüche.

Das erfindungsgemäße Verfahren zur Erstellung eines Bestrahlungsplans umfasst folgende Schritte:
a) Bereitstellen eines Abbildungsdatensatzes von dem zu bestrahlenden Zielobjekt,
b) Vorgeben eines im Abbildungsdatensatz abgebildeten Zielvolumens und einer zu erfüllenden Bedingung,
c) Durchführen eines Optimierungsverfahrens zur Ermittlung eines Parametersatzes, mit welchem eine Bestrahlungsanlage steuerbar ist,
   wobei bei dem Optimierungsverfahren der Parametersatz hinsichtlich der zu erfüllenden Bedingung unter Verwendung des Abbildungsdatensatzes optimiert wird und hierzu Rechenschritte durchgeführt werden, welche die Wirkung des Strahls auf das Zielobjekt kennzeichnen,
d) Erzeugen eines Bestrahlungsplanungsdatensatzes, indem der optimierte Parametersatz in dem Bestrahlungsplanungsdatensatz gespeichert wird,
   und wobei zusätzlich zu dem optimierten Parametersatz zumindest ein Teil der Zwischenergebnisse, welche bei dem Durchführen der Rechenschritte bei der Ermittlung des optimierten Parametersatzes ermittelt worden sind, gespeichert werden.

Bei dem erfindungsgemäßen Verfahren wird folglich eine Bestrahlungsplanung durchgeführt, wie sie in bekannter Weise durchgeführt wird, wie z.B. bei einer inversen Bestrahlungsplanung, bei der eine vorgegebene Zielfunktion optimiert wird. Hierbei wird der Parametersatz optimiert, der kennzeichnet, mit welchem Maschinenparametern die Bestrahlungsanlage zu steuern ist, um die Vorgabe eines Anwenders in Bezug auf ein zu bestrahlendes Zielvolumen und eine dort zu erreichende Dosisverteilung umzusetzen. Die zu erfüllende Bedingung wird normalerweise durch einen Anwender vorgegeben. Üblicherweise werden zu bestrahlende Zielvolumina, eine zu erreichende Soll-Dosisverteilung und zu schonende Zielorgane vorgegeben.

Zu dem Parametersatz eines Bestrahlungsplanungsdatensatzes gehören im Falle von gescannten Partikelstrahlen z.B. die Energie Eᵢ, die laterale Ablenkung (x, y)ᵢ, und die Anzahl der jeweils zu applizierenden Teilchen Nᵢ, und/oder der Fokus Fi wobei der Index i die einzelnen Rasterpunkte, also den Ort im Zielvolumen, kennzeichnet. Weitere Parameter können sein: die Focusgröße Fᵢ des Teilchenstrahls, die Intensität Iᵢ, mit der der Teilchenstrahl zu extrahieren ist, sowie etwaige Korrekturen ΔEᵢ, Δ (x, y)ᵢ, ΔNᵢ, ΔFᵢ der jeweiligen Parameter im Falle eines sich bewegenden Zielobjekts. Eine Steuerungsvorrichtung einer Anlage kann den Parametersatz auslesen und zur Steuerung der Anlage verwenden.

Das erfindungsgemäße Verfahren unterscheidet sich jedoch von herkömmlichen Verfahren zur Bestrahlungsplanung, indem der Bestrahlungsplanungsdatensatz nicht nur den Parametersatz enthält, der zur Steuerung der Bestrahlungsanlage verwendet wird und gegebenenfalls die mit diesem Parametersatz erzeugbare Dosisverteilung im Zielvolumen oder aus der Dosisverteilung abgeleitete Größen. Im neuen Bestrahlungsplanungsdatensatz werden ebenso Zwischenergebnisse hinterlegt, die bei der Durchführung der Berechnung der Bestrahlungsparameter bzw. des Parametersatzes ermittelt worden sind. Die Informationen sind im Bestrahlungsplanungsdatensatz in einer oder mehreren zueinander zugeordneten Speichereinheiten hinterlegt.

Die Zwischenergebnisse können unter anderem während der Berechnung der mit dem Parametersatz im Zielvolumen erzielbaren Dosisverteilung berechnet werden. Die Zwischenergebnisse stehen zwar im Zusammenhang mit der Wirkung des Strahls auf das Zielobjekt, die Zwischenergebnisse beschreiben jedoch die mit dem Strahl im Zielobjekt erzielbare Wirkung wie die erzielbare Dosisverteilung oder eine daraus ableitbare Größe noch nicht vollständig. Vielmehr sind die Zwischenergebnisse rechenaufwändige Teilergebnisse, aus welchen in weiteren, schnelleren Rechenschritten die erzielbare Dosisverteilung oder eine daraus abgeleitete Größe ermittelt werden kann.

Derartige abgeleitete Größen dienen üblicherweise der einfachen Beurteilung der Qualität einer Dosisverteilung. Hierzu gehören beispielsweise die mittlere (mean), die mediane (median), die fast-minimale und/oder die fastmaximale Dosis (bekannt unter den Bezeichnungen Dₘₑₐₙ, D_{50%} oder D_{median}, D_{98%} oder Dₙₑₐᵣ₋ₘᵢₙ, D_{2%} oder D_{near_max}) für die relevanten Zielvolumina VOI ("volume of interest") wie z.B. das CTV ("clinical target volume"), das PTV ("planned target volume") oder ein OAR ("organ at risk") bzw. einem PRV ("planning risk volume"). Ebenso gehören hierzu die Volumina oder die relativen Volumina V_{D} eines VOI, welche mindestens eine vorbestimmte Dosis D erhalten, oder die minimale Dosis Dᵥ, mit welcher das Volumen (oder relatives Volumens) eines VOI belegt wird, oder ein DVH (Dosis-Volumen-Histogramm).

Diese Zwischenergebnisse werden bei der Steuerung der Anlage nicht unmittelbar verwendet. Dies bedeutet, dass - wenn ein Bestrahlungsvorgang mit dem Bestrahlungsplanungsdatensatz durchgeführt wird - die Zwischenergebnisse nicht ausgelesen werden und bei der Steuerung der Bestrahlungsanlage eingesetzt werden.

Der Erfindung liegt jedoch die Erkenntnis zu Grunde, dass derartige Zwischenergebnisse bisher nicht Bestandteil eines Bestrahlungsplans waren, dass es aber vorteilhaft ist, diese Zwischenergebnisse in dem Bestrahlungsplanungsdatensatz zu hinterlegen. Die Zwischenergebnisse sind nämlich oftmals nur unter hohem rechnerischen Aufwand zu ermitteln. Auch wenn die Zwischenergebnisse prinzipiell wieder berechnet werden können, ist es vorteilhaft, diese Zwischenergebnisse nicht zu verwerfen, sondern im Bestrahlungsplanungsdatensatz zu hinterlegen. Dies eröffnet neue Möglichkeiten einer schnellen adaptiven Bestrahlungsplanung.

Zum Beispiel können die gespeicherten und damit unmittelbar zugänglichen Zwischenergebnisse dazu verwendet werden, eine schnellere Berechnung zu ermöglichen, wenn der Bestrahlungsplanungsdatensatz modifiziert werden soll. Auch wenn die Qualität des Bestrahlungsplans erneut überprüft werden soll, beispielsweise wenn eine erneute Dosisberechnung mit leicht modifizierten Randbedingungen durchgeführt werden soll, können diese Zwischenergebnisse verwendet werden, so dass sich die Berechnungszeit deutlich verkürzt.

Dem Optimierungsverfahren liegt üblicherweise ein Modell zugrunde, das bestimmte Annahmen über die Wechselwirkung des Partikelstrahls und der zu erzielenden Dosisverteilung in dem zu bestrahlenden Zielvolumen macht. Z.B. kann das zugrunde liegende Modell in eine Zielfunktion einfließen, die es bei der Durchführung des Optimierungsverfahrens zu optimieren gilt. Wenn das Optimierungsverfahren durchgeführt wird, werden bei der Berechnung des Parametersatzes auch Zwischenergebnisse erhalten, welche ebenso in das Modell einfließen.

Das erfindungsgemäße Verfahren zur Erstellung eines Bestrahlungsplansdatensatzes ist insbesondere bei der Partikeltherapie hilfreich, dort höchst insbesondere bei der Verwendung von Partikelstrahlen mit einer hohen Ordnungszahl wie beispielsweise Kohlenstoffionen. Dies rührt daher, dass die Interaktion von Partikeln mit einem zu bestrahlenden Zielvolumen komplexer ist als bei Photonen. Insbesondere, wenn die Bestrahlungsplanung für ein Scanverfahren durchzuführen ist, bei dem der Partikelstrahl nacheinander an eine Vielzahl von Rasterpunkten im Zielvolumen zu richten ist, kann die Bestrahlungsplanung aufgrund der vielen zu bestimmenden Parameter sehr rechenaufwändig sein.

Insbesondere können die Zwischenergebnisse Funktionswerte umfassen, die von zwei Ortsvariablen in dem zu bestrahlenden Zielobjekt abhängig sind. So kann die erste Ortsvariable z.B. Rasterpunkte i (i als Index für die Rasterpunkte) kennzeichnen, an denen eine Einzeldosis zu applizieren ist, und die zweite Ortsvariable Zielvoxel j des Zielvolumens (j als Index für die Zielvoxel).

Funktionswerte, die derart von zwei Ortsvariablen abhängen, können z.B. Dosiskorrelationen dᵢⱼ sein, die an einem Ort im Zielvolumen (z.B. an dem j-ten Zielvoxel) zur Gesamtdosis beitragen, verursacht durch den Teilstrahl, der auf einen anderen Ort im Zielvolumen zu richten ist (z.B. auf den i-ten Rasterpunkt). Andere derartige Funktionswerte können z.B. einen lateralen Abstand rᵢⱼ und einen Tiefenabstand zᵢⱼ zwischen einem ersten Ort (z.B. einem Rasterpunkt i) und einem zweiten Ort (z.B. einem Zielvoxel j) im Zielobjekt charakterisieren, wobei die Angabe des Tiefenabstandes die Reichweite des Partikelstrahls in dem zu bestrahlenden Zielobjekt berücksichtigt. Der Tiefenabstand ist folglich nicht der (einfach zu berechnende) geometrische Abstand zwischen den beiden Orten, sondern der um die Reichweiteninformation des Partikelstrahls korrigierte Abstand. Letzterer ist deutlich aufwändiger zu ermitteln, da er von den Gegebenheiten im Zielobjekt abhängt. Derartige Funktionswerte können in einer Tabelle hinterlegt werden.

Demgegenüber hängt der Parametersatz Ei, (x, y)ᵢ, Nᵢ, etc., bzw. die Dosisverteilung selbst, die bei einer herkömmlichen Bestrahlungsplanung bestimmt werden, nur von einer Ortsvariablen ab, z.B. von dem Rasterpunktindex i.

Die Zwischenergebnisse können auch ein durch den Partikelstrahl erzeugbares, ortsabhängiges Teilchenspektrum charakterisieren. Dies ist insbesondere bei der Bestrahlung mit Ionen, die schwerer sind als Protonen, vorteilhaft. In diesem Falle fragmentiert der Teilchenstrahl, und das entstehende Teilchenspektrum trägt seinerseits zu einer Dosisbelegung im Zielvolumen bei. Eine Dosisberechnung kann schneller durchgeführt werden, wenn ein bereits berechnetes Teilchenspektrum, das von den Gegebenheiten im Zielvolumen abhängt, abgespeichert und wieder verwendet wird.

Die Zwischenergebnisse können auch eine für den Partikelstrahl charakteristische Verteilung der relativen biologischen Wirksamkeit im Zielobjekt charakterisieren. Es ist bei der Erstellung des Bestrahlungsplans mitunter bereits aufwändig, die absorbierte, d.h. die durch den Partikelstrahl im Zielvolumen deponierte Dosis zu berechnen. Wenn man jedoch darüber hinaus die Wirkung der absorbierten Dosis berechnen will, das heißt die effektive Dosis, erfordert diese Berechnung weitere zeitaufwändige Rechenschritte. Im Allgemeinen kann dieser Unterschied zwischen absorbierter und effektiver Dosis mit einem Wichtungsfaktor beschrieben werden, der als relative biologische Wirksamkeit bekannt ist.

Aufgrund der Komplexität der Interaktion eines Partikelstrahls mit dem Zielvolumen ist es vorteilhaft, ein Modell zu Grunde zu legen, das eine ortsabhängige relative biologische Wirksamkeit verwendet. Die Berechnung der relativen biologischen Wirksamkeit erfordert dann einen hohen Rechenaufwand, da viele Abhängigkeiten von Parametern untereinander berücksichtigt werden müssen, zum Beispiel die Energie und die Größe des Partikelstrahls, das Material des zu bestrahlenden Zielvolumens, die Wechselwirkung und die Teilchensorte des Partikelstrahls, usw. abgespeichert in einem Bestrahlungsplanungsdatensatz erlaubt die Verteilung der relativen biologischen Wirksamkeit eine einfachere Neuberechnung/Modifikation einer bereits vorhandenen Bestrahlungsplanung. Wenn hierzu z.B. ein neuer Abbildungsdatensatz aufgezeichnet wird, kann unter Verwendung der bereits berechneten Verteilung der relativen biologischen Wirksamkeit eine Neuoptimierung des Parametersatzes schneller durchgeführt werden und führt im allgemeinen in weniger Iterationen zum Optimum als bei einer Planung ohne Vorinformationen.

Wenn nun der Bestrahlungsplan neu berechnet, optimiert und/oder variiert werden soll, können diese Zwischenergebnisse in die erneute Berechnung einfließen. Aufwändige Rechenschritte müssen somit nicht wiederholt werden, eine schnelle und effiziente Berechnung wird dadurch möglich. Dies eröffnet neue Möglichkeiten der Bestrahlung bzw. der Planung derselben, wie im Folgenden ausgeführt wird.

In einem erfindungsgemäßen Verfahren zur Anpassung einer Bestrahlungsplanung an eine Änderung eines Zielvolumens, bei welchem ein nach obigen Verfahren erzeugter Bestrahlungsplanungsdatensatz verwendet wird, werden folgende Schritte ausgeführt:
- Erfassen einer Änderung des Zielvolumens in Bezug auf seine Form/Lage im Vergleich zum Abbildungsdatensatz
- Berechnung einer Dosisverteilung für das Zielvolumen unter Verwendung der erfassten Änderung des Zielvolumens und der in dem Bestrahlungsplanungsdatensatz hinterlegten Zwischenergebnisse.

Auf diese Weise kann vergleichsweise schnell überprüft werden, ob ein Bestrahlungsplanungsdatensatz noch im Hinblick auf die Form- bzw. Lageänderung gültig ist, d.h. ob die ermittelten Parametersätze noch geeignet sind, um trotz einer etwaigen Änderung des Zielvolumens die gewünschte Dosisdeposition und den damit verbundenen gewünschten Bestrahlungserfolg zu erzielen.

Interfraktionäre Form- bzw. Lageänderungen können z.B. bei bestimmten Organen wie einer Prostata auftreten, aber auch bei Tumoren, deren Form/Lage durch Wundheilungsprozesse, Gewichtsverlust, etc. beeinflusst wird.

Die Änderung des Zielvolumens wird erfasst, indem ein weiterer Abbildungsdatensatz aufgezeichnet wird, in welchem das Zielvolumen mit geänderter Form und/oder Lage abgebildet ist und dieser weitere Abbildungsdatensatz mit dem Abbildungsdatensatz, der der ursprünglichen Bestrahlungsplanung zu Grunde gelegen hat, verglichen wird.

Da die Berechnung aufgrund der hinterlegten Zwischenergebnisse schneller durchgeführt werden kann, eröffnen sich neue Möglichkeiten: Beispielsweise kann der Bestrahlungsplanungsdatensatz unmittelbar vor einer Bestrahlungssitzung auf seine Gültigkeit hin verifiziert werden. Die Beurteilung, ob die neue Dosisverteilung noch der gewünschten Qualität entspricht, kann z.B. anhand von Dosis-Volumen-Histogrammen bewertet werden.

Falls notwendig, d.h. falls die erzielbare Dosisverteilung zu sehr von der ursprünglich erzielbaren und gewünschten Dosisverteilung abweicht, kann ein weiteres Optimierungsverfahren zur Modifikation des Parametersatzes durchgeführt werden, welches den Parametersatz hinsichtlich der Änderung des Zielvolumens optimiert. Das weitere Optimierungsverfahren kann die erfasste Änderung des Zielvolumens, den Bestrahlungsplanungsdatensatz und die in dem Bestrahlungsplanungsdatensatz hinterlegten Zwischenergebnisse verwenden.

Auch dies kann schneller durchgeführt werden als eine vollständig neue Bestrahlungsplanung, sodass es denkbar ist, das ganze unmittelbar vor einer geplanten Bestrahlungssitzung durchzuführen. Basierend auf der ursprünglichen Bestrahlungsplanung und den Zwischenergebnissen ist es denkbar, dass ein neues Optimum für den Parametersatz in vergleichsweise weniger Iterationsschritten unter Verwendung aktualisierter Parameter, z.B. einer Anpassung der Reichweite des Partikelstrahls und eine Anpassung der Nᵢ, gefunden werden kann.

Das weitere Optimierungsverfahren kann im Wesentlichen dem bei der Erstellung des Bestrahlungsplanungsdatensatzes eingesetzten Optimierungsverfahren entsprechen, nur dass nun die Rechenschritte, die zu den Zwischenschritten führen, nicht explizit ausgeführt werden müssen, da die bereits hinterlegten Zwischenergebnisse übernommen werden können.

Bei der Durchführung des weiteren Optimierungsverfahrens kann eine bereits im Zielvolumen applizierte Teildosis berücksichtigt werden, sodass insbesondere der Parametersatz für eine zu applizierende Restdosis optimiert wird. Insbesondere bei fraktionierten Bestrahlungen kann dann eine Anpassung der Bestrahlungsplanung schneller erfolgen. Auf diese Weise kann auch eine Abweichung einer Dosisdeposition, die bei vorausgegangenen Bestrahlungssitzungen aufgetreten sein kann, schnell und effizient ausgeglichen werden.

Der Abbildungsdatensatz und der weitere Abbildungsdatensatz können auch unterschiedlichen Phasen eines vierdimensionalen Abbildungsdatensatzes sein, die unterschiedlichen Bewegungsphasen eines sich bewegendes Zielvolumens zugeordnet sind. Ein derartiges Verfahren kann bei einem Tracking-Verfahren für die Optimierung von Kompensationsparametern beim Tracking verwendet werden. Wenn dazu die Bestrahlungsplanung einen vierdimensionalen Abbildungsdatensatz einsetzt, lässt sich die Bestrahlungsplanung insgesamt schneller durchführen. Es werden nämlich Zwischenergebnisse, die bei einer Referenzphase ermittelt worden sind, auch bei der Durchführung der Bestrahlungsplanung für eine andere Phase verwendet. Beispielsweise kann die Verteilung der relativen biologischen Wirksamkeit für eine Referenzphase bestimmt und dann auf andere Phasen übertragen werden. Hierzu kann es zu kleinen Abweichungen kommen gegenüber einer Bestrahlungsplanung, die bei jeder Phase getrennt die Verteilung der relativen biologischen Wirksamkeit erneut berechnet, die Abweichungen sind aber üblicherweise tolerierbar.

Bei einem erfindungsgemäßen Verfahren zur Optimierung einer Bestrahlungsplanung, können in einer ersten Phase
- mehrere Abbildungsdatensätze, in welchen ein zu bestrahlendes Zielvolumen mit unterschiedlicher Form und/oder Lage in einem Zielobjekt abgebildet ist, erfasst werden, und
- für jeden dieser Abbildungsdatensätze ein Bestrahlungsplanungsdatensatz mit einem der oben beschriebenen erfindungsgemäßen Verfahren erstellt werden.

In einer der ersten Phase nachfolgenden zweiten Phase kann
- ein weiterer Abbildungsdatensatz aufgezeichnet werden,
- der weitere Abbildungsdatensatz mit den mehreren Abbildungsdatensätzen hinsichtlich einer Ähnlichkeit verglichen werden,
- ein Abbildungsdatensatz aus den mehreren Abbildungsdatensätzen ausgewählt werden, welcher die größte Ähnlichkeit mit dem weiteren Abbildungsdatensatz aufweist,
- eine Änderung des Zielvolumens in Bezug auf seine Form/Lage zwischen dem ausgewählten Abbildungsdatensatz und dem weiteren Abbildungsdatensatz erfasst werden,
- ein weiteres Optimierungsverfahren zur Modifikation des Parametersatzes durchgeführt werden, welches den Parametersatz hinsichtlich der Änderung des Zielvolumens optimiert, wobei das weitere Optimierungsverfahren unter Verwendung der erfassten Änderung des Zielvolumens zwischen dem Abbildungsdatensatz und dem ausgewählten Abbildungsdatensatz und der in dem dem ausgewählten Abbildungsdatensatz zugeordneten Bestrahlungsplanungsdatensatz inklusive der hinterlegten Zwischenergebnisse durchgeführt wird.

Dieses Verfahren modifiziert Verfahren, bei denen die ursprüngliche Bestrahlungsplanung auf mehr als einem Volumendatensatz basiert. Dies wird z.B. bei der Bestrahlung der Prostata durchgeführt, um eine bessere Aussage über Lagevarianten des klinischen Zielvolumens treffen zu können. Auf diese Weise kann möglichst schnell und effizient die Bestrahlungsplanung einer möglichen Form- bzw. Lageänderung durchgeführt werden. Der einer aktuellen Lage am nächsten kommende ursprüngliche Bestrahlungsplan wird dann verwendet, um hiervon ausgehend die Bestrahlungsplanung weiter zu optimieren.

Die Bestrahlungsplanungseinrichtung umfasst eine Rechnereinheit mit einer Eingabeeinrichtung und einer Ausgabeeinrichtung, welche zur Durchführung eines der erfindungsgemäßen Verfahren ausgebildet ist.

Eine Bestrahlungsanlage, insbesondere Partikeltherapieanlage, umfasst eine derartige Bestrahlungsplanungseinrichtung und einer Steuerungsvorrichtung zur Steuerung der Bestrahlungsanlage aufgrund eines nach einem der erfindungsgemäßen Verfahren bereitgestellten und gegebenenfalls optimierten Bestrahlungsplanungsdatensatz.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung mit Weiterbildungen gemäß den Merkmalen der abhängigen Ansprüche werden anhand der folgenden Zeichnung näher erläutert ohne jedoch darauf beschränkt zu sein. Es zeigen:
Fig. 1 eine schematische Darstellung einer Partikeltherapieanlage mit verschiedenen Komponenten zur Überwachung der Bewegung eines zu bestrahlenden Zielvolumens, und
Fig. 2 ein Ablaufdiagramm einer Ausführungsform der Erfindung.

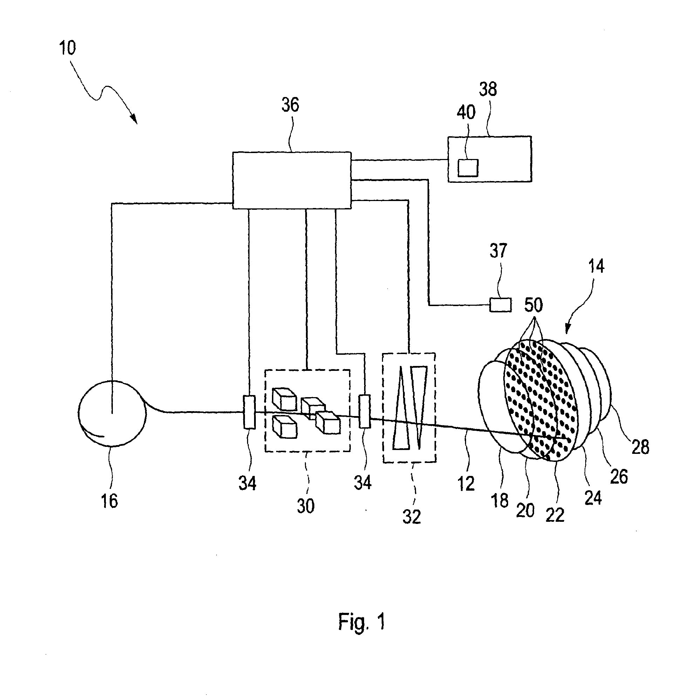

Fig. 1 zeigt in stark schematisierter Darstellung einen Aufbau einer Partikeltherapieanlage 10. Die Partikeltherapieanlage 10 wird zur Bestrahlung eines auf einer Positioniervorrichtung angeordneten Körpers mit einem Strahl aus Partikeln eingesetzt, der im Folgenden als Partikelstrahl 12 bezeichnet ist. Insbesondere kann als Zielvolumen 14 ein tumorerkranktes Gewebe eines Patienten mit dem Partikelstrahl 12 bestrahlt werden. Es ist ebenfalls vorgesehen, die Partikelstrahlanlage 10 zur Bestrahlung eines nicht-lebenden Körpers, insbesondere eines Wasserphantoms oder eines anderen Phantoms einzusetzen. Die Bestrahlung des Wasserphantoms kann beispielsweise zu Zwecken der Überprüfung und Verifizierung von Bestrahlungsparametern vor und/oder nach einer erfolgten Bestrahlung eines Patienten erfolgen. Es ist aber auch vorgesehen, andere Körper, insbesondere Versuchsaufbauten wie beispielsweise Zellkulturen oder Bakterienkulturen zu Forschungszwecken mit dem Partikelstrahl 12 zu bestrahlen. In allen Fällen kann es sich um bewegte oder ruhende Körper handeln.

Die Partikeltherapieanlage 10 weist typischerweise eine Beschleunigereinheit 16 auf, z.B. ein Synchrotron, ein Zyklotron oder einen sonstigen Beschleuniger, der einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat ein Partikelstrahl 12 einen Strahldurchmesser von 3-10 mm.

In dem zu bestrahlenden Zielvolumen 14 sind Schichten 18, 20, 22, 24, 26 und 28 angedeutet, welche Isoenergieschichten entsprechen. Eine Isoenergieschicht 18, 20, 22, 24, 26 oder 28 ist durch die Eindringtiefe des Partikelstrahls 12 bei einer bestimmten Energie des Partikelstrahls 12 gekennzeichnet. Jede Isoenergieschicht 18, 20, 22, 24, 26, 28 stellt bei dem hier gezeigten Beispiel einen Zielbereich innerhalb des Zielvolumens 14 dar, der im Rasterscanning-Verfahren bestrahlt werden soll.

Als Scanverfahren wird bevorzugt ein Rasterscan-Verfahren verwendet, bei dem ein Partikelstrahl 12 von Zielpunkt 50 zu Zielpunkt 50 geführt wird ohne zwangsläufige Abschaltung bei einem Übergang von einem Zielpunkt 50 zum nächsten. Es können auch Spotscan-Verfahren mit Abschaltung des Partikelstrahls zwischen den einzelnen Zielpunkten 50 oder andere ScanVerfahren wie beispielsweise kontinuierliche Scanverfahren eingesetzt werden, um den Zielbereich zu bestrahlen. In Fig. 1 sind einige Zielpunkt 50 der mittleren Isoenergieschicht 22 Zielvolumen 14 dargestellt, welche sukzessive mit dem Partikelstrahl 12 angefahren werden.

Der Partikelstrahl 12 wird in seiner lateralen Auslenkung mithilfe von Scan-Magneten 30 beeinflusst, d.h. in seiner Position senkrecht zu Strahlverlaufsrichtung, auch als x- und y-Richtung bezeichnet, abgelenkt. Weiterhin kann eine Energiemodulationsvorrichtung 32 vorgesehen sein, mit der die Energie des Partikelstrahls 12 schnell geändert werden kann, so dass die Eindringtiefe des Partikelstrahls 12 variiert werden kann. Auf diese Weise kann ein sich bewegendes Zielvolumen mit dem Partikelstrahl 12 "getrackt" werden oder als Beschleuniger 16 ein Zyklotron mit einer festen Energie verwendet werden.

Die Bestrahlungsanlage 10 weist ferner eine Ablaufsteuerung 36 und Detektoren 34 zur Überwachung der Strahlparameter auf. Die Anordnung der hier gezeigten Komponenten der Partikelstrahlanlage 10 ist lediglich beispielhaft. Auch Aufbauten einer anderen Anordnung sind denkbar.

Ferner kann die Bestrahlungsanlage 10 eine Vorrichtung zur Überwachung 37 einer möglichen Bewegung des Zielvolumens aufweisen. Die Ablaufsteuerung 36, also das Kontrollsystem der Anlage, steuert die einzelnen Komponenten der Anlage, wie beispielsweise den Beschleuniger 16, die Scan-Magnete 30 und sammelt Messdaten wie beispielsweise die Daten der Detektoren 34 zur Überwachung der Strahlparameter. Üblicherweise erfolgt die Steuerung basierend auf einem Bestrahlungsplanungsdatensatz 40, der mithilfe einer Bestrahlungsplanungseinrichtung 38 ermittelt und bereitgestellt wird.

In einer hier gezeigten Partikeltherapieanlage 10 können Ausführungsformen der Erfindung implementiert werden. Hierzu wird insbesondere die Bestrahlungsplanungseinrichtung 38 und je nach Ausführungsform auch die Ablaufsteuerung 36 entsprechend weitergebildet.

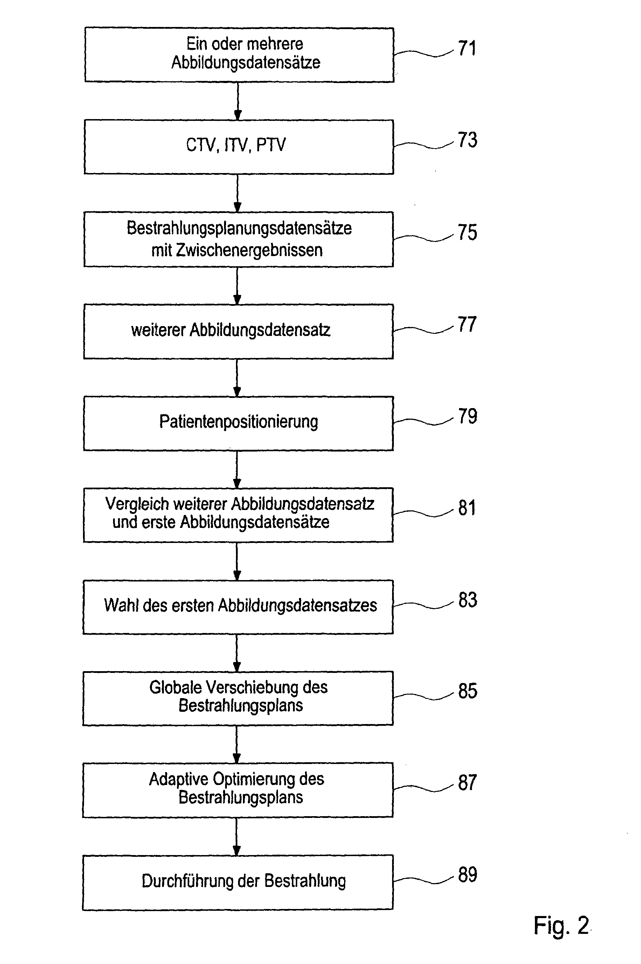

Fig. 2 zeigt schematisch einen Ablauf von Verfahrensschritten, der bei einer Ausführungsform des erfindungsgemäßen Verfahrens durchgeführt werden kann.

In einem ersten Schritt wird ein erster Abbildungsdatensatz, z.B. ein Computertomogramm (CT), aufgezeichnet. Der Abbildungsdatensatz liegt der Bestrahlungsplanung zu Grunde und bildet das zu bestrahlende Zielvolumen ab. Anstelle eines einzigen ersten Abbildungsdatensatzes können auch eine Vielzahl von ersten Abbildungsdatensätzen aufgezeichnet werden, in denen das Zielvolumen jeweils in leicht geänderter Lage und Forum abgebildet ist (Schritt 71). Dies ist vorteilhaft, um mögliche Lagevarianten des Zielvolumens bereits im Vorfeld während der Bestrahlungsplanungsphase zu erfassen.

In einem darauf folgenden Schritt werden in dem ersten Abbildungsdatensatz das Zielvolumen und etwaige Risikoorgane segmentiert. Die Segmentierung kann dabei manuell, halbautomatisch oder automatisch mit bekannten Segmentierungsalgorithmen durchgeführt werden. Anschließend werden die für die Bestrahlungsplanung relevanten Volumina wie das CTV, dass ITV ("internal target volume") und das PTV festgelegt (Schritt 73). Im Falle mehrerer erster Abbildungsdatensätze wird dieser Schritt für jeden der ersten Abbildungsdatensätze wiederholt.

Hierauf basierend wird die Bestrahlungsplanung durchgeführt (Schritt 75). Dabei wird der Parametersatz bestimmt, mit dem eine Anlage gesteuert wird, um zu erreichende Bestrahlungsvorgaben möglichst optimal umzusetzen. Im Falle einer Partikeltherapieanlage, wie sie in Fig. 1 dargestellt wurde, kann der Parametersatz auf einem Raster optimiert werden, das durch eine Anzahl von Iso-Energieschichten sowie von Rasterpunkten innerhalb der Iso-Energieschichten gekennzeichnet ist. Das Raster ist dabei ausreichend groß gewählt, um das PTV und alle denkbaren Verschiebungen des PTV zu erfassen. Bei dieser Optimierung wird eine Zielfunktion (auch Optimierungsfunktion genannt) minimiert. Eine derartige Zielfunktion ist z.B. in der Veröffentlichung von

Gemmel et al. "Biological dose optimization with multiple ion fields", 2008, Phys. Med. Biol. 53, 6991-7012,

Gleichung (2) angegeben. Für die Minimierung werden typischerweise Gradientenverfahren eingesetzt, d.h. es ist eine Berechnung der ersten Ableitung (Gradient) und je nach Verfahren auch der zweiten Ableitung (Hesse-Matrix) der Zielfunktion erforderlich.

Als Ergebnis wird einerseits der Parametersatz, welcher zur Steuerung der Anlage verwendet wird, ermittelt, wobei hierbei auch Zwischenergebnisse berechnet werden. Diese werden im Bestrahlungsplanungsdatensatz zusammen mit dem Parametersatz hinterlegt. Das Raster kann z.B. dazu verwendet werden, um die geometrischen Zusammenhänge zwischen Rasterpunkten und einzelnen Dosiswürfel als Teil der gespeicherten Zwischenschritte vorzuberechnen.

Als Zwischenergebnisse können z.B. gespeichert werden: ein ortsabhängiges Teilchenspektrum, eine ortsabhängige Verteilung der relativen biologischen Wirksamkeit, normierte Dosisbeiträge dᵢⱼ, welche kennzeichnen, wie stark die an einem Ort im Zielvolumen erzeugte Teildosis (z.B. auf den i-ten Rasterpunkt) auf ein j-tes Voxel wirkt (über die Gleichung Dⱼ = dᵢⱼ Nᵢ). Insbesondere für die Berechnung der relativen biologischen Wirksamkeit können wasseräquivalente Reichweiteninformationen zᵢⱼ und/oder Abstandsinformationen rᵢⱼ, gespeichert werden, welche die Reichweite des Partikelstrahls in dem zu bestrahlenden Zielobjekt zwischen einem ersten Ort (z.B. einem Rasterpunkt i) und einem zweiten Ort (z.B. einem Zielvoxel j) berücksichtigen.

Für eine weitere Beschleunigung des Rechenverfahrens können die Werte ζᵢₖ und zᵢⱼ und rᵢⱼ in die Gleichungen (14) der Veröffentlichung

Krämer and Scholz, "Rapid calculation of biological effects in ion radiotherapy", 2006, Phys. Med. Biol. 51, 1959-1970,

eingesetzt werden, um die Summen über die Teilchensorten T und die Energie E vorzuberechnen und zu speichern. Die Teilchensorten T und deren Energie E geben das durch den Partikelstrahl erzeugbare Teilchenspektrum an. Diese Zwischenergebnisse dienen dann zur Berechnung sowohl der biologisch effektiven Dosis (Gleichungen 16) als auch ihrer Ableitungen (Gleichungen 17), die zur Minimierung der Optimierungsfunktion notwendig sind.

Falls mehrere erste Abbildungsdatensätze aufgezeichnet worden sind, kann für jeden dieser ersten Abbildungsdatensätze eine Bestrahlungsplanung gemäß Schritt 75 durchgeführt werden.

Unmittelbar vor Beginn einer geplanten Bestrahlungssitzung, die Teil eine fraktionierten Bestrahlungsprogramms sein kann, wird ein weiterer Abbildungsdatensatz erstellt, mit dem die Bestrahlungsplanung und/oder die Lagerung des Patienten adaptiv angepasst werden kann (Schritt 77). Der weitere Abbildungsdatensatz kann beispielsweise ein volumetrischer Datensatz wie ein Cone-Beam CT sein, ein CT, das im Bestrahlungsraum durchgeführt wird, oder ein CT, das in einem anderen Raum unter Fixierung des Patienten in der Bestrahlungsposition durchgeführt wird.

Mithilfe des weiteren Abbildungsdatensatzes kann der Patient in der Bestrahlungsanlage passend positioniert werden (Schritt 79). Hierfür kann z.B. die in dem weiteren Datensatz üblicherweise gut dargestellte Knochenanatomie verwendet werden, um die Position des Patienten manuell zu justieren. Die Patientenpositionierung kann auch halbautomatisch bzw. automatisch erfolgen. Gegebenenfalls müssen die Sicherheitssäume um das Zielvolumen entsprechend gewählt werden, um etwaige Ungenauigkeiten bei den verschiedenen Positionierungsverfahren auszugleichen.

In einem weiteren Schritt erfolgt der Vergleich des weiteren Abbildungsdatensatzes mit dem oder den ersten Abbildungsdatensätzen, die der ursprünglichen Bestrahlungsplanung zu Grunde gelegen haben (Schritt 81). Bei diesem Vergleich kann ein geeignetes, bekanntes Ähnlichkeitsmaß eingesetzt werden. Beispielsweise kann ein Summe der quadratischen Abweichungen (SSD) aller Voxel berechnet werden, die einen Dosislevel >1% (oder >5% oder >10%) der Gesamtdosis belastet werden, ein SSD der Hounsfield-Units, eine Normalized Mutual Information, etc... um den Vergleich durchzuführen.

Nach erfolgtem Vergleich kann derjenige der ersten Abbildungsdatensätze bestimmt werden, der den geringsten Unterschied zu dem weiteren Abbildungsdatensatz aufweist (Schritt 83). Der zugehörige Bestrahlungsplanungsdatensatz wird geladen. Falls nur ein erster Abbildungsdatensatz erstellt worden ist, entfallen Schritt 81 und Schritt 83.

Darauf wird eine globale Verschiebung des Bestrahlungsplanungsdatensatzes - d.h. eine Verschiebung in lateraler Richtung und in Strahlrichtung - um einen Translationsvektor durchgeführt, der z. B. durch eine rigide Registrierung des ausgewählten ersten Abbildungsdatensatzes und des ursprünglichen Abbildungsdatensatzes bestimmt wird (Schritt 85). Hierdurch wird die Teilchenzahlverteilung des ursprünglichen Bestrahlungsplanungsdatensatzes unter Berücksichtigung der radiologischen Tiefe auf die Rasterpunkte innerhalb des neuen PTVs geschoben. Anstelle der rigiden Registrierung kann auch eine nicht-rigide Registrierung der beiden Abbildungsdatensätze zueinander durchgeführt werden kann. Der Abgleich der beiden Abbildungsdatensätze zueinander kann auch erfolgen, indem die bereits im ersten Abbildungsdatensatz segmentierten Strukturen im neuen Abbildungsdatensatz erneut segmentiert werden, z.B. manuell, halb-automatisch oder automatisch, und die segmentierten Strukturen zueinander in Beziehung gesetzt werden.

Anschließend kann der ausgewählte Bestrahlungsplan bzw. der zugehörige Parametersatz adaptiv optimiert werden, indem der bereits ermittelte Parametersatz unter Verwendung des weiteren Abbildungsdatensatzes und unter Verwendung des Bestrahlungsplanungsplans inklusive der gespeicherten Zwischenschritte im Hinblick auf die neuen verschobenen Rasterpunkte erneut optimiert wird (Schritt 87). Die Berechnung findet insgesamt schneller statt als bei einer vollständigen Neuberechnung. Der Parametersatz kann mit einer vergleichsweise geringen Anzahl an Iterationsschritten optimiert werden.

Die gewonnenen Ergebnisse können durch einen Arzt erneut überprüft werden, ehe unter Berücksichtung aller weiteren vorliegenden Umstände eine Bestrahlung gegebenenfalls freigegeben wird (Schritt 89). Die Erstellung des oder der Bestrahlungsplanungsdatensätze und die Verifikation bzw. Neuberechnung findet also im Vorfeld einer geplanten Bestrahlung statt. Ob die geplante Bestrahlung allerdings tatsächlich durchgeführt wird, wird durch einen Arzt unter Berücksichtung weiterer Umstände entschieden.

### Bezugszeichenliste

- 10: Partikeltherapieanlage
- 11: Partikelstrahl
- 14: Zielvolumen
- 16: Beschleunigereinheit
- 18, 20, 22, 24, 26, 28: Isoenergieschicht
- 30: Scan-Magnete
- 32: Energiemodulationsvorrichtung
- 34: Detektor
- 36: Ablaufsteuerung
- 37: Bewegungsüberwachungsvorrichtung
- 38: Bestrahlungsplanungsvorrichtung
- 40: Bestrahlungsplan
- 50: Rasterpunkte
- 71: Schritt 71
- 73: Schritt 73
- 75: Schritt 75
- 77: Schritt 77
- 79: Schritt 79
- 81: Schritt 81
- 83: Schritt 83
- 85: Schritt 85
- 87: Schritt 87
- 89: Schritt 89

## Patentansprüche

1. Verfahren zur Erstellung eines Bestrahlungsplans einer Partikeltherapieanlage mit einem Partikelstrahl (12) mit Protonen, Kohlenstoffionen oder anderen Ionen, umfassend folgende Schritte:
a) Bereitstellen eines Abbildungsdatensatzes von dem mit dem Partikelstrahl (12) mit den Protonen, den Kohlenstoffionen oder den anderen Ionen zu bestrahlenden Zielobjekt, welches ein Zielvolumen (14) umfasst,
b) Vorgeben eines im Abbildungsdatensatz abgebildeten Zielvolumens (14) und einer zu erfüllenden Bedingung,
c) Durchführen eines Optimierungsverfahrens zur Ermittlung eines Parametersatzes, mit welchem die Partikeltherapieanlage (10) steuerbar ist, wobei bei dem Optimierungsverfahren der Parametersatz hinsichtlich der zu erfüllenden Bedingung unter Verwendung des Abbildungsdatensatzes optimiert wird und hierzu Rechenschritte durchgeführt werden, welche die Wirkung des Partikelstrahls (12) auf das Zielobjekt kennzeichnen, **gekennzeichnet durch**:
d) Erzeugen eines Bestrahlungsplanungsdatensatzes (40), indem der optimierte Parametersatz in dem Bestrahlungsplanungsdatensatz (40) gespeichert wird, wobei zusätzlich zu dem optimierten Parametersatz zumindest ein Teil der Zwischenergebnisse, welche bei dem Durchführen der Rechenschritte bei der Ermittlung des optimierten Parametersatzes ermittelt worden sind, in dem Bestrahlungsplanungsdatensatz zusammen mit dem Parametersatz gespeichert werden, wobei die Zwischenergebnisse Teilergebnisse sind, aus welchen in weiteren Rechenschritten die erzielbare Dosisverteilung oder eine daraus abgeleitete Größe ermittelt werden kann, und diese Zwischenergebnisse nicht zur unmittelbaren Verwendung bei der Steuerung der Partikeltherapieanlage sind, und
wobei diese Zwischenergebnisse in eine erneute Berechnung einfließen, wenn der Bestrahlungsplan neu berechnet werden soll.

2. Verfahren nach Anspruch 1, wobei die erste Ortsvariable Rasterpunkte (50) kennzeichnet, an denen eine Einzeldosis zu applizieren ist, und die zweite Ortsvariable Zielvoxel des Zielobjekts oder Rasterpunkte, an denen eine Einzeldosis zu applizieren ist, kennzeichnet.

3. Verfahren nach Anspruch 1 oder 2, wobei die Funktionswerte eine Dosisdeposition an einem Ort im Zielobjekt, verursacht durch einen Teilstrahl, der auf einen anderen Ort im Zielvolumen (14) zu richten ist, charakterisieren.

4. Verfahren nach einem der Ansprüche 1 bis 3, wobei die Funktionswerte einen Abstand zwischen einem ersten Ort und einem zweiten Ort im Zielobjekt charakterisieren, welcher die Reichweite eines Partikelstrahls (12) in dem zu bestrahlenden Zielobjekt berücksichtigt.

5. Verfahren nach einem der Ansprüche 1 bis 4, wobei die Zwischenergebnisse ein durch einen Partikelstrahl (12) erzeugbares Teilchenspektrum charakterisieren.

6. Verfahren nach einem der Ansprüche 1 bis 5, wobei die Zwischenergebnisse eine für einen Partikelstrahl (12) charakteristische Verteilung der relativen biologischen Wirksamkeit im Zielobjekt charakterisieren.

7. Verfahren zur Anpassung einer Bestrahlungsplanung einer Partikeltherapieanlage an eine Änderung eines Zielvolumens (14), bei welchem ein Bestrahlungsplanungsdatensatz (40) nach einem der Ansprüche 1 bis 6 verwendet wird, umfassend folgende Schritte:
- Erfassen einer Änderung des Zielvolumens (14) in Bezug auf seine Form und/oder Lage im Vergleich zum Abbildungsdatensatz
- Berechnung einer Dosisverteilung für das Zielvolumen (14) unter Verwendung der erfassten Änderung des Zielvolumens (14) und der in dem Bestrahlungsplanungsdatensatz (40) hinterlegten Zwischenergebnisse.

8. Verfahren nach Anspruch 7, wobei ein weiteres Optimierungsverfahren zur Modifikation des Parametersatzes durchgeführt wird, welches den Parametersatz hinsichtlich der Änderung des Zielvolumens (14) optimiert, wobei das weitere Optimierungsverfahren unter Verwendung der erfassten Änderung des Zielvolumens (14) und der in dem Bestrahlungsplanungsdatensatz (40) hinterlegten Zwischenergebnisse durchgeführt wird.

9. Verfahren zur Optimierung einer Bestrahlungsplanung, bei welchem
in einer ersten Phase
- mehrere Abbildungsdatensätze, in welchen ein zu bestrahlendes Zielvolumen (14) mit unterschiedlicher Form und/oder Lage in einem Zielobjekt abgebildet ist, erfasst werden und,
- für jeden dieser Abbildungsdatensätze ein Bestrahlungsplanungsdatensatz (40) mit einem Verfahren nach einem der Ansprüche 1 bis 6 erstellt wird,
und bei welchem in einer der ersten Phase nachfolgenden zweiten Phase
- ein weiterer Abbildungsdatensatz aufgezeichnet wird,
- der weitere Abbildungsdatensatz mit den mehreren Abbildungsdatensätzen hinsichtlich einer Ähnlichkeit verglichen wird,
- ein Abbildungsdatensatz aus den mehreren Abbildungsdatensätzen ausgewählt wird, welcher die größte Ähnlichkeit mit dem weiteren Abbildungsdatensatz aufweist,
- eine Änderung des Zielvolumens (14) in Bezug auf seine Form und/oder Lage zwischen dem ausgewählten Abbildungsdatensatz und dem weiteren Abbildungsdatensatz erfasst wird,
- ein weiteres Optimierungsverfahren zur Modifikation des Parametersatzes durchgeführt wird, welches den Parametersatz hinsichtlich der Änderung des Zielvolumens optimiert, wobei das weitere Optimierungsverfahren unter Verwendung der erfassten Änderung des Zielvolumens zwischen dem Abbildungsdatensatz und dem ausgewählten Abbildungsdatensatz und der in dem dem ausgewählten Abbildungsdatensatz zugeordneten Bestrahlungsplanungsdatensatz (40) hinterlegten Zwischenergebnisse durchgeführt wird.

10. Bestrahlungsplanungseinrichtung (38), umfassend eine Rechnereinheit mit einer Eingabeeinrichtung und einer Ausgabeeinrichtung, welche zur Durchführung eines Verfahrens nach einem der Ansprüche 1 bis 9 ausgebildet ist.

11. Partikeltherapieanlage (10), mit einer Bestrahlungsplanungseinrichtung (38) nach Anspruch 10 und einer Steuerungsvorrichtung (36) zur Steuerung der Partikeltherapieanlage aufgrund eines nach einem Verfahren nach einem der Ansprüche 1 bis 9 hergestellten Bestrahlungsplanungsdatensatzes (40).

## Claims

1. Method for drawing up an irradiation plan for a particle therapy installation comprising a particle beam (12) comprising protons, carbon ions or other ions, comprising the following steps:
a) providing an image data record of the target object to be irradiated, comprising a target volume (14), by the particle beam (12) comprising the protons, the carbon ions or the other ions,
b) predetermining a target volume (14) imaged in the image data record and a condition to be satisfied,
c) performing an optimization method for establishing a parameter set by means of which the particle therapy installation (10) is controllable, with the parameter set being optimized during the optimization method in respect of the condition to be satisfied using the image data record and calculation steps being performed to this end, which calculation steps characterize the effect of the particle beam (12) on the target object, **characterized by**:
d) producing an irradiation planning data record (40) by virtue of the optimized parameter set being stored in the irradiation planning data record (40), with, in addition to the optimized parameter set, at least some of the intermediate results, which were established when performing the calculation steps when establishing the optimized parameter set, being stored in the irradiation planning data record together with the parameter set, with the intermediate results being partial results from which the obtainable dose distribution or a variable derived therefrom can be established in further calculation steps and these intermediate results not being for direct use in the control of the particle therapy installation, and these intermediate results being included in a new calculation if the irradiation plan is to be recalculated.

2. Method according to Claim 1, wherein the first spatial variable characterizes grid points (50) at which an individual dose is to be applied and the second spatial variable characterizes target voxels of the target object or grid points at which an individual dose is to be applied.

3. Method according to Claim 1 or 2, wherein the functional values characterize a dose deposition at a location in the target object, caused by a partial beam, which is to be directed to a different location in the target volume (14).

4. Method according to one of Claims 1 to 3, wherein the functional values characterize a distance between a first location and a second location in the target object, which takes into account the reach of a particle beam (12) in the target object to be irradiated.

5. Method according to one of Claims 1 to 4, wherein the intermediate results characterize a particle spectrum producible by a particle beam (12).

6. Method according to one of Claims 1 to 5, wherein the intermediate results characterize a distribution, characteristic for a particle beam (12), of the relative biological effectiveness in the target object.

7. Method for adapting irradiation planning for a particle therapy installation to a change in a target volume (14), in which an irradiation planning data record (40) according to one of Claims 1 to 6 is used, comprising the following steps:
- registering a change in the target volume (14) in relation to the shape and/or position thereof compared to the image data record,
- calculating a dose distribution for the target volume (14) using the registered change in the target volume (14) and the intermediate results stored in the irradiation planning data record (40).

8. Method according to Claim 7, wherein a further optimization method is performed for modifying the parameter set, said optimization method optimizing the parameter set in respect of the change in the target volume (14), the further optimization method being performed using the registered change in the target volume (14) and the intermediate results stored in the irradiation planning data record (40).

9. Method for optimizing irradiation planning, in which, in a first phase,
- a plurality of imaging data records, in which a target volume (14) to be irradiated is respectively imaged with a different shape and/or position in a target object, are registered and,
- for each one of these image data records, an irradiation planning data record (40) is drawn up using a method according to one of Claims 1 to 6, and in which, in a second phase following the first phase,
- a further image data record is recorded,
- the further image data record is compared to the plurality of image data records in respect of a similarity,
- an image data record which has the greatest similarity with the further image data record is selected from the plurality of image data records,
- a change in the target volume (14) in respect of the shape and/or position thereof is registered between the selected image data record and the further image data record,
- a further optimization method for modifying the parameter set is performed, which optimizes the parameter set in respect of the change in the target volume, the further optimization method being performed using the registered change in the target volume between the image data record and the selected image data record and the intermediate results stored in the irradiation planning data record (40) assigned to the selected image data record.

10. Irradiation planning apparatus (38), comprising a computer unit comprising an input apparatus and an output apparatus, embodied to perform a method according to one of Claims 1 to 9.

11. Particle therapy installation (10) comprising an irradiation planning apparatus (38) according to Claim 10 and a control device (36) for controlling the particle therapy installation on the basis of an irradiation planning data record (40) produced according to a method according to one of Claims 1 to 9.

## Revendications

1. Procédé pour élaborer un programme de traitement par rayonnement d'une installation de thérapie par particules avec un faisceau de particules (12) comportant des protons, des ions de carbone ou d'autres ions, comprenant les étapes suivantes:
a) mise à disposition d'un jeu de données de reproduction de l'objet cible comportant un volume cible (14) et devant être irradié avec le faisceau de particules (12) comportant les protons, les ions de carbone ou les autres ions,
b) prédéfinition d'un volume cible (14) reproduit dans le jeu de données de reproduction et d'une condition à remplir,
c) exécution d'un procédé d'optimisation pour déterminer un jeu de paramètres qui permet de commander l'installation de thérapie par particules (10), sachant que le jeu de paramètres est optimisé lors du procédé d'optimisation en ce qui concerne la condition à remplir, en utilisant le jeu de données de reproduction, et que l'on exécute à cet effet des étapes de calcul qui caractérisent l'action du faisceau de particules (12) sur l'objet cible, **caractérisé par**
d) la production d'un jeu de données de programmation de traitement par rayonnement (40) en mémorisant le jeu de paramètres optimisé dans le jeu de données de programmation de traitement par rayonnement (40), sachant que l'on mémorise dans le jeu de données de programmation de traitement par rayonnement, en plus du jeu de paramètres optimisé, conjointement avec le jeu de paramètres, au moins une partie des résultats intermédiaires qui ont été obtenus lors de l'exécution des étapes de calcul lors de la détermination du jeu de paramètres optimisé, sachant que les résultats intermédiaires sont des résultats partiels à partir desquels on peut déterminer, au cours d'étapes de calcul supplémentaires, la distribution de dose pouvant être obtenue, ou une grandeur dérivée de celle-ci, et que ces résultats intermédiaires ne sont pas destinés à une utilisation directe lors de la commande de l'installation de thérapie par particules, et sachant que ces résultats intermédiaires entrent dans un nouveau calcul lorsqu'il doit y avoir un nouveau calcul du programme de traitement par rayonnement.

2. Procédé selon la revendication 1, selon lequel la première variable de lieu caractérise des points de trame (50) auxquels il faut appliquer une dose individuelle, et la deuxième variable de lieu caractérise des voxels cibles de l'objet cible ou des points de trame auxquels il faut appliquer une dose individuelle.

3. Procédé selon la revendication 1 ou 2, selon lequel les valeurs de fonction caractérisent un dépôt de dose à un lieu dans l'objet cible, provoqué par un faisceau partiel qui doit être dirigé sur un autre lieu dans le volume cible (14).

4. Procédé selon l'une des revendications 1 à 3, selon lequel les valeurs de fonction caractérisent une distance entre un premier lieu et un deuxième lieu dans l'objet cible, qui prend en compte la portée d'un faisceau de particules (12) dans l'objet cible à irradier.

5. Procédé selon l'une des revendications 1 à 4, selon lequel les résultats intermédiaires caractérisent un spectre de particules pouvant être produit par un faisceau de particules (12).

6. Procédé selon l'une des revendications 1 à 5, selon lequel les résultats intermédiaires caractérisent une distribution de l'efficacité biologique relative dans l'objet cible, qui est caractéristique d'un faisceau de particules (12).

7. Procédé pour adapter un programme de traitement par rayonnement d'une installation de thérapie par particules à une modification du volume cible (14), selon lequel on utilise un jeu de données de programmation de traitement par rayonnement (40) d'après l'une des revendications 1 à 6, comprenant les étapes suivantes :
- détection d'une modification du volume cible (14) par rapport à sa forme et/ou sa position comparée au jeu de données de reproduction
- calcul d'une distribution de dose pour le volume cible (14), en utilisant la modification détectée du volume cible (14) et les résultats intermédiaires enregistrés dans le jeu de données de programmation de traitement par rayonnement (40).

8. Procédé selon la revendication 7, selon lequel on exécute un autre procédé d'optimisation pour modifier le jeu de paramètres, qui optimise le jeu de paramètres en ce qui concerne la modification du volume cible (14), le procédé d'optimisation supplémentaire étant mis en oeuvre en utilisant la modification détectée du volume cible (14) et
les résultats intermédiaires enregistrés dans le jeu de données de programmation de traitement par rayonnement (40).

9. Procédé pour optimiser un programme de traitement par rayonnement, selon lequel,
au cours d'une première phase,
- plusieurs jeux de données de reproduction sont recueillis, dans lesquels est reproduit un volume cible (14) à irradier, ayant une forme et/ou une position variable dans un objet cible, et
- pour chacun de ces jeux de données de reproduction, un jeu de données de programmation de traitement par rayonnement (40) est établi avec un procédé d'après l'une des revendications 1 à 6,
et selon lequel, au cours d'une deuxième phase succédant à la première phase,
- un jeu de données de reproduction supplémentaire est enregistré,
- le jeu de données de reproduction supplémentaire est comparé avec la pluralité de jeux de données de reproduction en ce qui concerne une ressemblance,
- un jeu de données de reproduction est sélectionné parmi la pluralité de jeux de données de reproduction, qui présente la plus grande ressemblance avec le jeu de données de reproduction supplémentaire,
- une modification du volume cible (14) par rapport à sa forme et/ou sa position entre le jeu de données de reproduction sélectionné et le jeu de données de reproduction supplémentaire est détectée,
- un procédé d'optimisation supplémentaire est exécuté pour modifier le jeu de paramètres, qui optimise le jeu de paramètres par rapport à la modification du volume cible, le procédé d'optimisation supplémentaire étant mis en oeuvre en utilisant la modification détectée du volume cible entre le jeu de données de reproduction et le jeu de données de reproduction sélectionné, et les résultats intermédiaires enregistrés dans le jeu de données de programmation de traitement par rayonnement (40) associé au jeu de données de reproduction sélectionné.

10. Dispositif de programmation de traitement par rayonnement (38), comprenant une unité d'ordinateur qui est dotée d'un dispositif d'entrée et d'un dispositif de sortie et est conçue pour exécuter un procédé selon l'une des revendications 1 à 9.

11. Installation de thérapie par particules (10), comprenant un dispositif de programmation de traitement par rayonnement (38) selon la revendication 10 et un dispositif de commande (36) pour commander l'installation de thérapie par particules sur la base d'un jeu de données de programmation de traitement par rayonnement (40) établi selon un procédé d'après l'une des revendications 1 à 9.
